# EUROPEAN PATENT SPECIFICATION

(11) **EP 2 409 672 B1**
(45) Date of publication and mention of the grant of the patent: **25.10.2017**
(21) Application number: 11174681.4
(22) Date of filing: 20.07.2011
(51) Int. Cl.: A61F 5/56

(54) **Oral fixation device and fixing method using the same**
Orale Fixiervorrichtung und Fixierverfahren
Dispositif de fixation buccale et procédé de fixation l'utilisant

(30) Priority: 21.07.2010 TW 99124051; 23.12.2010 US 977293
(43) Date of publication of application: 25.01.2012
(73) Proprietor: Industrial Technology Research Institute, Chutung Hsinchu 31040 (TW); National Taiwan University Hospital, Taipei 100 (TW)
(72) Inventor: Huang, Chen-Ning, Dayuan Township 337 Taoyuan County (TW); Lin, Chen-Liang, 600 Chiayi City (TW); Chen, Yunn-Jy, 106 Taipei City (TW)
(74) Representative: dompatent von Kreisler Selting Werner - Partnerschaft von Patent- und Rechtsanwälten mbB

(56) References cited:
- EP-A2- 1 192 928
- DE-U1- 9 201 026
- KR-A- 20100 096 049
- US-A- 2 643 652
- US-A- 5 950 624
- US-A- 5 979 449
- US-A1- 2008 105 268
- US-A1- 2008 210 244

## Description

The disclosure relates in general to a fixing device and a fixing method using the same, and more particularly to an oral fixation device and an oral fixing method using the same.
A dental oral appliance to reduce or eliminate snoring is known from US 2008/0210244. The appliance covers the inside (lingual) of the upper or lower teeth and has an open palate. Retention for the appliance is provided by an occlusion coverage of the upper or lower teeth. A raised incisor ramp extends from the incisal tip of the incisors toward the lingual, or posterior ramps, separate the posterior teeth to open the airway. A trans-palatal bar, which extends from the inside (lingual) of the right molars to the inside of the left molars inhibits the upward and backward movement of the tongue. An anterior tongue restrainer which is attached to this transpalatal bar at one end and the front of the appliance at the other end aids in inhibiting and restraining the upward and backward movement of the tongue.
A suction device adaptable to the tongue and palate, which allows for an increased ventilation space in the rear area of the nasal cavities is known from EP 1192928 A2.
KR 10 - 2010 - 0096049 discloses an oral fixation device.

US 2,643,652 discloses a mouthpiece mouthpiece for application to the upper teeth, comprising a U-shape trough portion adapted to accommodate the upper teeth of individuals within a substantial range of variations in mouth size and shape; and a soft flexible palatal membrane spanning the lingual area defined by said U-shape trough portion, said palatal membrane terminating in a rear edge adapted to engage the hard palatal region of an individual just anterior of the junction of the hard and soft palates and being sufficiently thin to conform to the contour of the roof of the mouth of a wearer upon removal of air from between said palatal .membrane and said roof.

US 2008/0105268 A1 discloses an airway maintenance apparatus containing an upper-jaw side attachment to be attached to the upper jaw side in the oral cavity, and a lower-jaw side attachment provided separately from the upper-jaw side attachment, and to be attached to the lower jaw side in the oral cavity, the lower-jaw side attachment being connected to the upper-jaw side attachment via a stepless adjusting device capable of moving the lower-jaw side attachment in a stepless manner in the direction of drawing the lower jaw forward relative to the upper-jaw side attachment 1.

How to modify the current oral fixation device as a device which can fix the teeth, the tongue and other oral organs has become an imminent task for the industries.

The disclosure is directed to an oral fixation device according to claim 1. The arch board and the U-shaped brace are combined, so that the user can fix the teeth as well as the tongue with the oral fixation device.

According to a first aspect of the present disclosure, an oral fixation device is provided. The oral fixation device includes a U-shaped brace and an arch board. The arch board filling the space between the tongue and the maxillary of an oral cavity is the arch board having a central part and a connecting part to the inner wall. The arch board is connected to an inner wall of the U-shaped brace, wherein the central part of the arch board is thicker than the connecting part, and the maximum thickness of the arch board is larger than 2 mm. The U-shaped brace has a U-shaped upper bite channel and a U-shaped lower bite channel, the front end of the U-shaped upper bite channel is separated from the inner wall by a first distance, the front end of the U-shaped lower bite channel is separated from the inner wall by a second distance, and the second distance is larger than the first distance.

The above and other aspects of the disclosure will become better understood with regard to the following detailed description of the non-limiting embodiment(s). The following description is made with reference to the accompanying drawings.
FIG. 1 is a 3-D diagram of an oral fixation device according to an embodiment of the disclosure;
FIG. 2 is a back view of an oral fixation device;
FIG. 3 is an upper view of an oral fixation device;
FIG. 4 is a cross-sectional view of the oral fixation device of FIG. 3 viewed along a cross-sectional line 4-4';
FIG. 5 is a flowchart of using an oral fixation device; and
FIG. 6 is a user interposing the oral fixation device between the tongue and the maxillary.

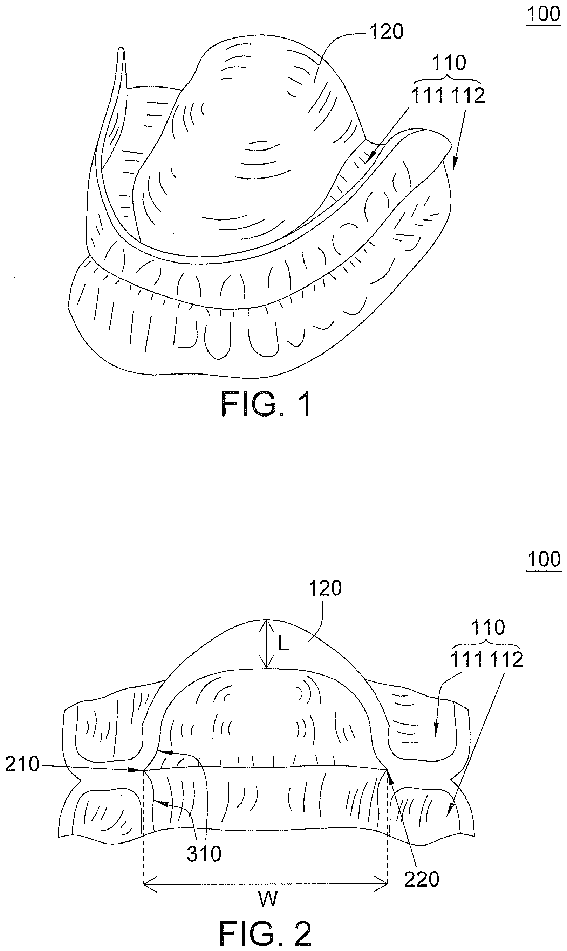

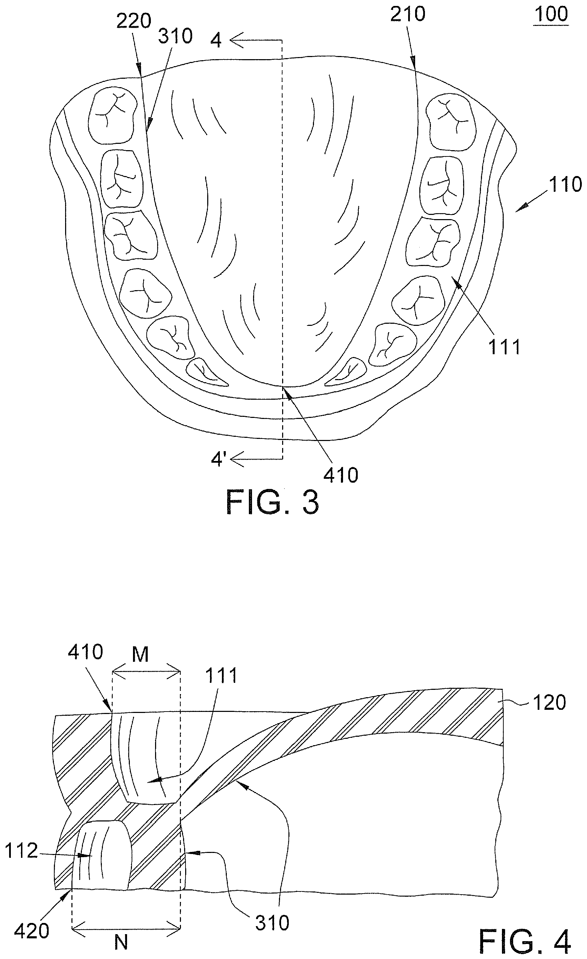

Referring to FIGS. 1∼4. FIG. 1 is a 3-D diagram of an oral fixation device 100 according to an embodiment of the disclosure. FIG. 2 is a back view of an oral fixation device 100. FIG. 3 is an upper view of an oral fixation device 100. FIG. 4 is a cross-sectional view of the oral fixation device of FIG. 3 viewed along a cross-sectional line 4-4'.

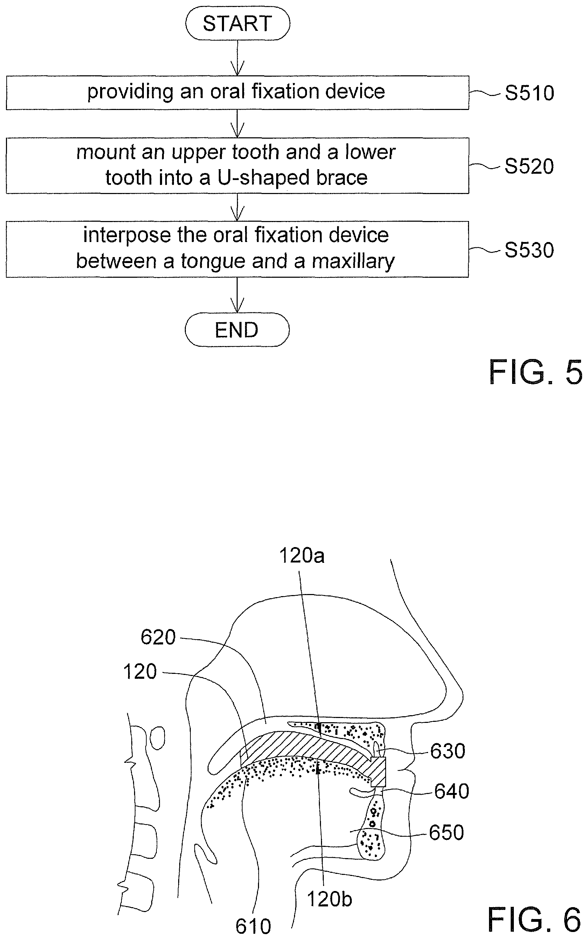

The oral fixation device 100 of the present embodiment of the disclosure includes a U-shaped brace 110 and an arch board 120. The U-shaped brace 110 is used for mounting an upper tooth 630 (illustrated in FIG. 6) and a lower tooth 640 (illustrated in FIG. 6) of an oral cavity. The arch board 120 with non-uniform thickness is connected to an inner wall 310 of the U-shaped brace 110 and interposed between a tongue 610 (illustrated in FIG. 6) and a maxillary 620 (illustrated in FIG. 6). The central part of the arch board 120 is thicker than the connecting part. For example, the thickness of the arch board 120 progressively increases from the connecting part towards the central part. In an embodiment of the disclosure, the U-shaped brace can be a bite block.

As indicated in FIG. 2, a maximum thickness L of the arch board 120 being substantially equal to the distance between a tongue 610 and a maxillary 620 is larger than 2 mm but smaller than 15 mm or is tailor made to fit the user's needs. For example, if the user is a child, the maximum thickness L of the arch board 120 can range between 2∼8 mm, so that the arch board 120 can hold the tongue 610 and the cause of respiratory tract obstruction can be removed.

As indicated in the oral device 100 of FIG. 4, the U-shaped brace 110 further has a U-shaped upper bite channel 111 and a U-shaped lower bite channel 112. The front end 410 of the U-shaped upper bite channel 111 is separated from the inner wall 310 by a first distance M. The front end 420 of the U-shaped lower bite channel 112 is separated from the inner wall 310 by a second distance N. The second distance N is larger than the first distance M. In other words, the U-shaped lower bite channel 112 is substantially located in front of the U-shaped upper bite channel 111, and when the user wears the oral fixation device 100, the mandible 650 (illustrated in FIG. 6) will be slightly advanced forwards, so that the cross area of the respiratory tract can be enlarged.

In addition, the arch board 120 is interposed between a tongue 610 and a maxillary 620. To stabilize the position of the user's tongue, the arch board 120 fills the space between the tongue 610 and the maxillary 620 of the user's oral cavity and characterizes a non-uniform distribution of thickness. Again, referring to FIG. 2, the maximum thickness L of the arch board 120 is located at the central part of the arch board 120. That is, the arch board 120 is thicker at the central part but thinner at the two sides. In the oral cavity of a human, the clearance between the tongue 610 and the maxillary 620 is also wider at the central part and narrower at the two sides. When the user wears the oral fixation device 100, the above design enables the user's tongue to be firmly fixed under the arch board 120 of the oral fixation device 100.

Referring to FIG. 5 and FIG. 6. FIG. 5 is a flowchart of using the oral fixation device 100. FIG. 6 is the user interposing the oral fixation device 100 between a tongue 610 and a maxillary 620. Firstly, the method begins at step S510, the oral fixation device 100 is provided.

Next, the method proceeds to step S520, an upper tooth 630 and a lower tooth 640 are mounted into a U-shaped brace 110. As indicated in FIG. 6, the upper tooth 630 and the lower tooth 640 are firmly fixed in the U-shaped brace 110, and the biting position of the upper tooth and the lower tooth is adjusted, so that the front end 410 of the U-shaped upper bite channel 111 is separated from the inner wall 310 by a first distance M, and the front end 420 of the U-shaped lower bite channel 112 is separated from the inner wall 310 by a second distance N which is larger than the first distance M. In other words, the U-shaped lower bite channel 112 is substantially located in front of the U-shaped upper bite channel 111 as indicated in FIG. 4.

Then, the method proceeds to step S530, the oral fixation device 100 is interposed between the tongue 610 and the maxillary 620, and the thickness of the arch board 120 is adjusted according to the clearance between the tongue 610 and the maxillary 620, so that an upper surface 120a of the arch board 120 substantially contacts the maxillary 620, and a lower surface 120b of the arch board 120 substantially contacts the tongue 610. It is shown in FIG. 6 that when the upper surface 120a and the lower surface 120b of the arch board 120 substantially contact the maxillary 620 and the tongue 610 respectively, the tongue 610, being squeezed by the arch board 120 which is fixed under the maxillary 620, is fixed under the arch board 120.

Thus, when the user's tooth bites the U-shaped brace 110, the arch board 120 is concurrently squeezed by both the U-shaped brace 110 and the maxillary 620, the arch board 120 fixes the tongue 610 from above, front, the left and the right to assure that the tongue 610 is firmly fixed.

According to the design of the oral fixation device 100 of the present embodiment of the disclosure, the front end 410 of the U-shaped upper bite channel 111 is separated from the inner wall 310 by a first distance M, and the front end 420 of the U-shaped lower bite channel 112 is separated from the inner wall 310 by a second distance N which is larger than the first distance M, so the oral fixation device 100 can maintain the open of the respiratory tract as the mandible 650 (illustrated in FIG. 6) is advanced forward. Moreover, since the arch board 120 can hold the tongue 610, the cause of respiratory tract obstruction can be removed. In addition, the arch board 120 fills the space surrounding the tongue 610, supports the tongue 610 and prevents it from falling due to gravity when the user lies down. Thus, the user's respiratory tract will not be obstructed. Therefore, the oral fixation device 100 of the present embodiment of the disclosure at least has the features of "maintaining the open of the respiratory tract", "removing the cause of respiratory tract obstruction" and "keeping the tongue at a natural position lest the tongue might fall due to gravity".

The tongue 610 is kept at a natural position and can be easily stabilized by the oral fixation device 100 with only a small amount of effort, so the user will not feel uncomfortable even after wearing the oral fixation device 100 for a long duration. Since the oral fixation device 100 can fix the upper tooth 630 and the lower tooth 640, the user's mouth will not be opened to breathe when he is asleep with the oral fixation device 100 in his oral cavity. The oral fixation device 100 of the present embodiment of the disclosure further has the feature of "causing no discomfort even after a long duration of use".

Experiments were undertaken with five testees from July 2009 to December 2009 to assess the effect of oral fixation device of present disclosure. At first, these five testees were checked that they are not the following patients. (1) Non-obstructive sleep apnea patients. (2) Patients having other disease about sleeping and breathing except obstructive sleep apnea syndrome (OSAS). (3) Patients having systemic disease, such as rheumatoid arthritis. (4) Patients having Temporo-Mandibular disorder. (5) Patients having periodontal disease or toothache, or people who can't use braces.

The experiment was started at the step of making testees to undergo polysomnography (PSG) examination, recording the age, body mass index (BMI), and neck circumference data of the testees, and taking a lateral cephalometric photography for testees. Then dentist produced a general oral appliance and an oral fixation device of the present disclosure separately for the testees after modeling the oral cavity. The testees wore the general oral appliances three months and underwent PSG examination to assess the effect of wearing the general oral appliances. And then the testees wore the oral fixation device of the present disclosure three months and underwent PSG examination to assess the effect of wearing the oral fixation device. Table 1 shows the result of the experiment. Wherein the "AHI" means the apnea hypopnea index (the severity of OSAS)

**Table 1: The result of the experiment**

| | AHI of 1^{st} PSG | AHI of 2^{nd} PSG | AHI of 3^{rd} PSG | Improvement AHI |
|---|---|---|---|---|
| Testee | Before Test | Wear General Oral Appliance | Wear the Oral Fixation Device | ΔAHI |
| A | 14.8 | 16.5 | 1.5 | 89.56% |
| B | 116.3 | 82.2 | 67.7 | 41.79% |
| C | 57.6 | 58.0 | 45.6 | 20.83% |
| D | 9.2 | 18.9 | 1.3 | 85.87% |
| E | 25.1 | 51.8 | 10.4 | 58.57% |

It is clear that testee wearing the oral fixation device of present disclosure would reduce the severity of OSAS. Thus, in contrast to the general oral appliances, the oral fixation device of the disclosure is more suitable for being used as an oral appliance for preventing respiratory tract obstruction, which may cause sleep apnea.

## Claims

1. An oral fixation device, comprising:
a U-shaped brace (110); and
an arch board (120) connected to an inner wall (310) of the U-shaped brace (110) and arched in a central part of the arch board (120), wherein the central part of the arch board (120) is thicker than the connecting part, and the maximum thickness (L) of the arch board (120) is larger than 2 mm, wherein the U-shaped brace (110) has a U-shaped upper bite channel (111) and a U-shaped lower bite channel (112), **characterised in that** a front end (410) of the U-shaped upper bite channel is separated from the inner wall (310) by a first distance (M), a front end (420) of the U-shaped lower bite channel (112) is separated from the inner wall (310) by a second distance (N), and the second distance (N) is larger than the first distance (M), such that the front end (420) of the U-shaped lower bite channel (112) is located in front of the front end (410) of the U-shaped upper bite channel (111) to advance a mandible (650) of a user forward.

2. The oral fixation device according to claim 1, wherein the maximum thickness (L) of the arch board (120) is smaller than 15 mm.

3. The oral fixation device according to claim 1, wherein the maximum thickness (L) is located at the central part of the arch board (120).

4. The oral fixation device according to claim 1, wherein the maximum thickness (L) is larger than 2 mm but smaller than 8 mm.

5. The oral fixation device according to claim 1, wherein the thickness (L) of the arch board (120) increases progressively from the connecting part towards the central part.

## Patentansprüche

1. Orale Fixiervorrichtung mit:
einer U-förmigen Zahnklammer (110); und
einer Bogenplatte (120), die mit einer Innenwand (310) der U-förmigen Zahnklammer (110) verbunden ist und in einem zentralen Teil der Bogenplatte (120) gekrümmt ist, wobei der zentrale Teil der Bogenplatte (120) dicker als der Verbindungsteil ist und die maximale Dicke (L) der Bogenplatte (120) größer als 2 mm ist, wobei die U-förmige Zahnklammer (110) einen U-förmigen oberen Bisskanal (111) und einen U-förmigen unteren Bisskanal (112) aufweist,
**dadurch gekennzeichnet, dass** ein Vorderende (410) des U-förmigen oberen Bisskanals um eine erste Distanz (M) von der Innenwand (310) beabstandet ist, ein Vorderende (420) des U-förmigen unteren Bisskanals (112) um eine zweite Distanz (N) von der Innenwand (310) beabstandet ist, und die zweite Distanz (N) größer als die erste Distanz (M) ist, derart, dass das Vorderende (420) des U-förmigen unteren Bisskanals (112) vor dem Vorderende (410) des U-förmigen oberen Bisskanals (111) angeordnet ist, um eine Mandibel (650) eines Benutzers nach vorne zu verlagern.

2. Orale Fixiervorrichtung nach Anspruch 1, bei der die maximale Dicke (L) der Bogenplatte (120) kleiner als 15 mm ist.

3. Orale Fixiervorrichtung nach Anspruch 1, bei der die maximale Dicke (L) an dem zentralen Teil der Bogenplatte (120) existiert.

4. Orale Fixiervorrichtung nach Anspruch 1, bei der die maximale Dicke (L) größer als 2 mm, jedoch kleiner als 8 mm ist.

5. Orale Fixiervorrichtung nach Anspruch 1, bei der die Dicke (L) der Bogenplatte (120) von dem Verbindungsteil zu dem zentralen Teil hin progressiv zunimmt.

## Revendications

1. Dispositif de fixation buccale, comprenant :
un appareil dentaire en forme de U (110) ; et
une planche arquée (120) raccordée à une paroi intérieure (310) de l'appareil dentaire en forme de U (110) et arquée dans une partie centrale de la planche arquée (120), dans lequel la partie centrale de la planche arquée (120) est plus épaisse que la partie de raccordement, et l'épaisseur maximale (L) de la planche arquée (120) est supérieure à 2 mm, dans lequel l'appareil dentaire en forme de U (110) a un canal de morsure supérieur en forme de U (111) et un canal de morsure inférieur en forme de U (112), **caractérisé en ce qu'**une extrémité avant (410) du canal de morsure supérieur en forme de U est séparée de la paroi intérieure (310) d'une première distance (M), une extrémité avant (420) du canal de morsure inférieur en forme de U (112) est séparée de la paroi intérieure (310) d'une seconde distance (N), et la seconde distance (N) est supérieure à la première distance (M), de sorte que l'extrémité avant (420) du canal de morsure inférieur en forme de U (112) soit située à l'avant de l'extrémité avant (410) du canal de morsure supérieur en forme de U (111) pour faire avancer une mandibule (650) d'un utilisateur vers l'avant.

2. Dispositif de fixation buccale selon la revendication 1, dans lequel l'épaisseur maximale (L) de la planche arquée (120) est inférieure à 15 mm.

3. Dispositif de fixation buccale selon la revendication 1, dans lequel l'épaisseur maximale (L) est située au niveau de la partie centrale de la planche arquée (120).

4. Dispositif de fixation buccale selon la revendication 1, dans lequel l'épaisseur maximale (L) est supérieure à 2 mm mais inférieure à 8 mm.

5. Dispositif de fixation buccale selon la revendication 1, dans lequel l'épaisseur (L) de la planche arquée (120) augmente progressivement de la partie de raccordement vers la partie centrale.
